Europäisches Patentamt

European Patent Office

Office européen des brevets

(19)

(11) **EP 0 193 881 B1**

(12) **EUROPÄISCHE PATENTSCHRIFT**

(45) Veröffentlichungstag und Bekanntmachung des Hinweises auf die Patenterteilung:
**28.08.1996  Patentblatt 1996/35**

(51) Int Cl.⁶: **G01N 33/74**, G01N 33/577, C07K 16/26, C12P 21/08, C12N 15/00, C12N 5/00 // (C12P21/00, C12R1:91)

(21) Anmeldenummer: **86102590.6**

(22) Anmeldetag: **28.02.1986**

(54) **Verfahren und Reagenz zur Bestimmung des luteinisierenden Hormons sowie hierzu geeignete monoklonale Antikörper**

Method and reagent for the determination of a luteinizing hormone, as well as the appropriate monoclonal hormone

Méthode et réactif pour la détermination de l'hormone lutéinisante ainsi que l'anticorps monoclonal approprié

(84) Benannte Vertragsstaaten:
**AT BE CH DE FR GB IT LI LU NL SE**

(30) Priorität: **06.03.1985  DE 3507848**

(43) Veröffentlichungstag der Anmeldung:
**10.09.1986  Patentblatt 1986/37**

(73) Patentinhaber: **BOEHRINGER MANNHEIM GMBH 68298 Mannheim (DE)**

(72) Erfinder:
- **Hübner-Parajsz, Christa, Dr.**
  **D-8132 Tutzing (DE)**
- **Schetters, Hartmut, Dr.**
  **D-8056 Neufahrn (DE)**
- **Lenz, Helmut, Dr.**
  **D-8132 Tutzing (DE)**
- **Erler, Klaus, Dr.**
  **D-8134 Pöcking (DE)**

(74) Vertreter: **Huber, Bernhard, Dipl.-Chem. et al Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**81635 München (DE)**

(56) Entgegenhaltungen:
**FR-A- 2 480 782**       **GB-A- 2 111 201**

- **CLINICAL CHEMISTRY, Band 28, Nr. 9, 1982; H.G. WADA et al., Seiten 1862-1866**
- **ANNUAL REVIEW OF BIOCHEMISTRY, Band 50, 1981; J.G. PIERCE, Seiten 465, 476-477**
- **ANNUAL OF CLINICAL BIOCHEMISTRY, 1984; W.M. HUNTER et al., Seiten 275-283**
- **CLINICA CHIMICA ACTA, Band 133, 1983; M. SOOS et al., Seiten 263-274**
- **ENDOCRINOLOGY, Band 117, 1985; Seiten 2428-2434**
- **JOURNAL OF IMMUNOLOGICAL METHODS, Band 51, 1982, Elsevier Biomedical Press; M. SOOS et al., Seiten 57-68**
- **WIENER KLINISCHE WOCHENSCHRIFT, Jg. 97, Nr. 14, 1985; P. BERGER, Seiten 573-581**
- **T 906/91 (25 April 1995)**
- **NATURE, Band 256, 07 August 1975; G. KÖHLER et al., Seiten 495-497**

**Beschreibung**

Die Bestimmung des luteinisierenden Hormons (LH) in Körperflüssigkeiten, beispielsweise Urin, Serum und Plasma wird hauptsächlich angewendet, um den endokrinologischen Status von Hypothalamus, Hypophyse und Gonaden beurteilen zu können. Diese Untersuchungen dienen vor allem der Differenzialdiagnose von Hypogonadismus, Infertilität usw. Daneben wird die LH-Bestimmung eingesetzt, um den Ovulationszeitpunkt bei der Induktion von Schwangerschaften zu ermitteln.

In allen diesen Fällen liegen die Serumwerte im physiologischen Bereich. Darüber hinaus wird die Konzentration von LH im Serum auch lediglich zu dem Zweck gemessen, um eine Aussage über die biologische Wirksamkeit dieses Hormons zu erhalten. Von diagnostischer Bedeutung ist daher im wesentlichen die Kenntnis des Serumspiegels des nativen Hormons.

Die physiologische Konzentration von humanem LH im Serum liegt in den folgenden Bereichen:

| | |
|---|---|
| Männer | 4 - 24 mIU/ml |
| Frauen vor Menopause, Zyklus | 10 - 20 mIU/ml |
| Frauen Ovulationspeak | 80 - 100 mIU/ml |
| Frauen nach Menopause | 80 - 150 mIU/ml |

Für die Bestimmung von LH eignen sich vor allem immunologische Testverfahren, bei denen das Hormon als Antigen mit einem oder mehreren dagegen gerichteten Antikörpern bestimmt wird. Die Antikörpergewinnung mit diesen Polypeptid-Hormonen ist mit Schwierigkeiten verbunden, da alle Polypeptid-Hormone schlecht immunogen sind. Wegen der Homologie zwischen LH und anderen Glykoprotein-Hormonen, wie beispielsweise dem Follikel stimulierenden Hormon (FSH), Thyreotropin stimulierenden Hormon (TSH) und humanen Choriongonadotropin (nCG), ist es sehr schwierig, spezifische Antikörper gegen eines dieser Hormone zu gewinnen. Ein gegen eines dieser Glykoprotein-Hormone gerichteter Antikörper weist üblicherweise mehr oder weniger Kreuzreaktivität mit den übrigen Glykoprotein-Hormonen auf.

Im Clinica Chemica Acta 133 (1983), Seite 263 - 274 werden monoklonale Antikörper gegen LH beschrieben, die mit TSH und hCG zu 10 % und mit FSH zu 3 % kreuzreagieren. Aus GB-A-2 111 201 ist ebenfalls ein monoklonaler Antikörper gegen LH bekannt. Dieser reagiert jedoch ebensogut mit hCG. Ein monoklonaler Antikörper der spezifisch gegen LH gerichtet ist und keine Kreuzreaktivität mit den übrigen Glykoprotein-Hormonen aufweist, ist bisher nicht bekannt. Deshalb ist es derzeit nicht möglich, LH immunologisch zu bestimmen, ohne daß andere Glykoprotein-Hormone mehr oder weniger mit erfaßt werden.

Aufgabe der Erfindung war es, ein neues immunologisches Verfahren und Reagenz bereit zu stellen, mit dessen Hilfe LH auch in Gegenwart von anderen Glykoprotein-Hormonen spezifisch erfaßt werden kann. Gelöst wird diese Aufgabe durch das erfindungsgemäße immunologische Verfahren und Reagenz.

Gegenstand der Erfindung ist daher ein immunologisches Verfahren zur Bestimmung des luteinisierenden Hormons, bei dem mindestens ein monoklonaler Antikörper eingesetzt wird, der spezifisch gegen LH gerichtet ist und mit anderen Glykoprotein-Hormonen weniger als 3 % kreuzreagiert, sowie ein hierfür geeignetes Reagenz.

Als immunologische Bestimmungmethode eignen sich im Prinzip alle gängigen Immuno-Assays, wie Radio-Immuno-Assay, Enzym-Immuno-Assay, Fluoreszenz-Immuno-Assay usw. Ferner sind sämtliche Verfahrensvarianten, wie kompetitiver Immuno-Assay, Sandwich-Verfahren usw., anwendbar. Zur Markierung eignen sich die für die jeweilige Bestimmungsmethode üblichen Mittel. So werden bei einem Radio-Immuno-Assay Radioisotope, beispielsweise $^{125}J$, zur Markierung verwendet. Für einen Enzym-Immuno-Assay sind sämtliche, hierfür üblicherweise eingesetzten Enzyme, beispielsweise Peroxidase oder β-Galaktosidase, geeignet. Für einen Fluoreszenz-Immuno-Assay sind die üblichen fluoreszierenden Gruppen als Marker brauchbar. Einzelheiten dieser verschiedenen Testmethoden und Verfahrensvarianten sind dem Fachmann bekannt. Vorteilhaft sind Testvarianten, bei denen mindestens zwei monoklonale Antikörper eingesetzt werden, die gegen verschiedene antigene Determinanten des LH gerichtet sind und von denen mindestens einer mit anderen Glykoprotein-Hormonen weniger als 3 % Kreuzreagiert.

Zur Bestimmung von LH hat sich als besonders zweckmäßig das Sandwich-Verfahren erwiesen, bei dem das zu bestimmende Antigen mit einem trägergebundenen und einem markierten Antikörper in Kontakt gebracht wird. Für ein solches Bestimmungsverfahren kann beispielsweise ein erfindungsgemäßer, spezifischer monoklonaler Antikörper an die Festphase gebunden werden. Dieser wird in dem ersten Inkubationsschritt mit der Probe, die zu bestimmendes LH sowie im allgemeinen andere Glykoprotein-Hormone enthält, inkubiert. Hierbei wird von dem spezifischen Antikörper selektiv LH gebunden. Nach dem üblichen Waschschritt wird mit dem markierten Antikörper inkubiert. Dieser muß nicht notwendigerweise spezifisch gegen LH gerichtet sein, sondern kann auch mit anderen Glykoprotein-Hormonen kreuzreagieren.

Das Verfahren Kann auch mit einem unspezifischen trägergebundenen Antikörper durchgeführt werden. Es ist dann jedoch erforderlich, daß als markierter Antikörper ein erfindungsgemäßer, spezifisch gegen LH gerichteter Antikörper eingesetzt wird.

Varianten des Sandwich-Verfahrens sind ebenfalls zur Bestimmung von LH geeignet. So kann beispielsweise zunächst mit einem nicht markierten, löslichen Antikörper und dem markierten Antikörper ein löslicher Sandwich-Komplex gebildet werden. Dieser wird anschließend mit Hilfe eines trägergebundenen Antikörpers, der gegen den Fcγ-Teil des nicht markierten löslichen Antikörpers gerichtet ist, unlöslich gemacht. Bei dieser Verfahrensvariante muß mindestens der nicht markierte, lösliche Antikörper ein erfindungsgemäßer Antikörper sein. Der markierte Antikörper wird dabei vorzugsweise im Überschuß eingesetzt.

Das Wesentliche der vorliegenden Erfindung ist darin zu sehen, daß es überraschenderweise gelang, für diese immunologischen Verfahren monoklonale Antikörper bereit zu stellen, die spezifisch gegen LH gerichtet sind, und die daher eine spezifische Bestimmung von LH ermöglichen.

Ein weiterer Gegenstand der Erfindung sind daher monoklonale Antikörper gegen LH, deren Kreuzreaktivität mit anderen Glykoprotein-Hormonen weniger als 3 % beträgt.

Zur Gewinnung der erfindungsgemäßen monoklonalen Antikörper werden Versuchstiere, beispielsweise Mäuse, mit LH immunisiert. Zur Immunisierung wird das Immunogen beispielsweise in Kombination mit einem Adjuvans in üblicher Weise verabreicht. Als Adjuvans wird Aluminiumhydroxid zusammen mit Bordetella pertussis eingesetzt. Die Immunisierung erfolgt vorzugsweise über mehrere Monate mit mindestens vier Immunisierungen in 4 - 6wöchigem Abstand (Injektion intraperitoneal).

Aus so immunisierten Tieren werden B-Lymphozyten gewonnen, die mit einer permanenten Myelomzellinie fusioniert werden. Die Fusionierung erfolgt nach dem bekannten Verfahren von Köhler und Milstein (Nature 256, 1975, S. 495 - 497). Die hierbei gebildeten Primärkulturen von Hybridzellen werden in üblicher Weise, z. B. unter Verwendung eines handelsüblichen Zellsorters oder durch "limiting dilution" kloniert. Es werden jeweils die Kulturen weiterverarbeitet, die in einem geeigneten Testverfahren, beispielsweise einem Enzym-Immuno-Assay (ELISA-Verfahren), positiv gegen LH und negativ bzw. nur wenig mit den anderen Glykoprotein-Hormonen reagieren. Man erhält so mehrere Hybridoma-Zellinien, die die erfindungsgemäßen monoklonalen Antikörper produzieren. Nach bekannten Methoden können diese Zellinien kultiviert und die von ihnen produzierten monoklonalen Antikörper isoliert werden.

Beispielhaft für auf diese Weise gewonnen Zellinien seien angeführt:

Klon 369     (NCACC 84122001) und
Klon 799     (NCACC 84122005).

Die Zellinien sind unter der jeweils angegebenen Nummer bei der Hinterlegungsstelle NCACC (National Collection of Animal Cell Cultures) hinterlegt.

Die so gewonnenen monoklonalen Antikörper zeichnen sich dadurch aus, daß sie eine sehr hohe Affinität (Affinitätskonstante in der Größenordnung von $10^9$ bis $10^{11}$ l/mol) gegen LH besitzen und mit anderen Glykoprotein-Hormonen weniger als 3 % kreuzreagieren. Besonders bevorzugte monoklonale Antikörper weisen eine Kreuzreaktivität gegenüber anderen Glykoprotein-Hormonen, wie hCG, FSH und TSH, von weniger als 1 %, ganz besonders bevorzugt von weniger als 0,1 % auf. Zur Bestimmung der Affinität und der Kreuzreaktivität mit anderen Hormonen stehen die üblichen, dem Fachmann bekannten Verfahren zur Verfügung.

Die erfindungsgemäßen monoklonalen Antikörper eignen sich hervorragend dazu, in einer Probe, beispielsweise Serum oder Plasma, das Hormon LH in Gegenwart anderer Glykoprotein-Hormone spezifisch zu bestimmen. Für diese Bestimmungsverfahren können die monoklonalen Antikörper als solche oder Fragmente hiervon, welche die entsprechenden immunologischen Eigenschaften aufweist, beispielsweise Fab-Fragmente, verwendet werden. Unter dem Begriff "monoklonale Antikörper" werden daher sowohl die vollständigen Antikörper als auch die Fragmente verstanden.

Die Erfindung wird durch die folgenden Beispiele näher erläutert:

Beispiel 1

Gewinnung von monoklonalen Antikörpern gegen LH

Balb/c-Mäuse, 8-12 Wochen alt, werden mit 100 µg hLH (Firma Immunex), an Aluminiumhydroxid und Bordetella pertussis adsorbiert, intraperitoneal immunisiert. Nach 6 Wochen werden drei weitere Immunisierungen in 4wöchigem Abstand durchgeführt. Dabei werden jeweils 50 µg LH, an Aluminiumhydroxid und Bordetella pertussis adsorbiert, intraperitoneal verabreicht.

Ungefähr vier Monate nach der letzten Immunisierung wird fusioniert Vier Tage und drei Tage vor der Fusionierung wird jeweils noch einmal mit 100 µg LH/PBS (= phosphate buffered saline), intraperitoneal bzw. intravenös immunisiert.

Zur Fusion werden in Anlehnung an Galfré, Methods in Enzymology, 73 (1981), Seite 3, einmal $10^8$ Milzzellen einer immunisierten Maus mit 2 x $10^7$ Myelomzellen (P3x63Ag8-653, ATCC-CRL 8375) gemischt und anschließend 10 Minuten zentrifugiert (300 g, 4° C). Die Zellen werden noch einmal mit BSS (= balanced salt solution) gewaschen und bei 400 g zentrifugiert. Der Überstand wird entfernt. Das Zellsediment wird mit 1 ml 50 %iger PEG-Lösung (MG 4000, Merck) versetzt. Danach wird bei Raumtemperatur 5 ml RPMI 1640-Medium (RPMI = Rosewell Parker Memory Institut) ohne fötalem Kälberserum (FKS), anschließend noch einmal 5 ml RPMI 1640-Medium, mit 10 % FKS langsam zugetropft, mit Medium auf 50 ml aufgefüllt und 10 Minuten bei 400 g zentrifugiert. Die sedimentierten Zellen werden in RPMI 1640-Medium mit 10 % FKS aufgenommen. Je 2 x $10^5$ Milzzellen werden auf 24-well-Zellkulturplatten (Firma Nunc) eingesät. Zu jeder Kultur werden 1 x $10^5$ Milzzellen oder 5 x $10^4$ Peritoneal-Exudat-Zellen als Futterzellen zugefügt. Am folgenden Tag wird Hypoxanthin-Azaserin-Selektionsmedium (100 mM Hypoxanthin, 1 µg/ml Azaserin) zugegeben.

Nach ca. 7 - 10 Tagen werden bereits viele Klone sichtbar. Der Überstand der Primärkulturen wird nach einem in Beispiel 2 beschriebenen ELISA-Verfahren getestet. Primärkulturen, die Antigen-spezifische Antikörper enthalten, werden mit Hilfe eines fluoreszenzaktivierenden Zellsorters auf 96-well-Zellkulturplatten (Firma Nunc) weiter Kloniert. Als Futterzellen dienen 1 x $10^4$ Peritoneal-Exudat-Zellen oder 2 x $10^4$ Milzzellen pro 96er-well der Kultur.

Auf diese Weise konnten die beiden Hybridoma-Zellinien Klon 369 und Klon 799 isoliert werden, die bei der Hinterlegungsstelle NCACC (= National Collection of Animal Cell Cultures) unter den Hinterlegungsnummern

    NCACC 84122001       (= Klon 369) und

    NCACC 84122005       (= Klon 799)

hinterlegt worden sind.

Zur Erzeugung von Ascites werden 5 x $10^6$ Hybrid-Zellen intraperitoneal Mäusen gespritzt, die zuvor 1 - 2mal mit 0,5 ml Pristan vorbehandelt worden sind. Eine bis drei Wochen danach kann aus den Mäusen Ascites-Flüssigkeit gewonnen werden. Hieraus können in üblicher Weise die Antikörper isoliert werden. Diese monoklonalen Antikörper sind spezifisch gegen LH gerichtet und zeigen keine bzw. nur eine geringe Kreuzreaktivität mit anderen Glykoprotein-Hormonen. Sie werden im folgenden mit MAK 369 (von Klon 369) bzw. MAK 799 (von Klon 799) bezeichnet.

Die beiden monoklonalen Antikörper gehören der Subklasse IgGl/K an. Sie besitzen die folgenden Affinitäten:

$$MAK\ 369 = 7.9\ x\ 10^{10}\ l/mol$$

$$MAK\ 799 = 9.5\ x\ 10^{11}\ l/mol.$$

Zur Ermittlung der Affinitäten werden Kompetitionskurven mit dem homologen Antigen nach Scatchard (Ann. N. Y. Acad. Sci. 51 (1949) Seite 660) ermittelt und ausgewertet. Die hierfür erforderlichen Messungen werden analog Beispiel 2 durchgeführt.

Beispiel 2

Screening-Test auf Antikörper gegen LH

Um die Anwesenheit und Spezifität von Antikörpern gegen LH im Serum immunisierter Mäuse oder in dem Kulturüberstand der Hybridzellen oder in Ascites zu erkennen, wird ein ELISA-Verfahren als Testprinzip verwendet: Mikrotiterplatten werden mit 1 µg LH/ml Beschichtungspuffer (0,2 M Natriumcarbonat/-Bicarbonat, pH 9,3-9,5) bei 37 °C über Nacht beschichtet. Es wird 10 Minuten mit 0,9 % Natriumchloridlösung und 1 % Albuminlösung nachbehandelt. Anschließend wird mit 0,9 % Natriumchloridlösung gewaschen. Anschließend wird bei 37 °C eine Stunde mit 100 µl Probe inkubiert und erneut mit 0,9 % Natriumchloridlösung gewaschen. Es folgt eine weitere Inkubation (eine Stunde 37 °C) mit 100 - 150 mU/ml eines Schaf-anti-Maus-IgG-Peroxidase-Konjugats. Nach einem erneuten Waschschritt mit 0,9 % Natriumchlorid wird die Peroxidaseaktivität in üblicher Weise bestimmt (beispielsweise mit ABTS, 30 Minuten bei Raumtemperatur, abgelesen wird die Extinktionsdifferenz, ΔmE, bei 405 nm).

Der ELISA-Test kann auch wie folgt durchgeführt werden:

Die Mikrotiterplatten werden zunächst mit einem Schaf-anti-Maus-IgG beschichtet (20 - 30 µg/ml Beschichtungspuffer, eine Stunde bis über Nacht, 37° C). Danach wird wie oben beschrieben, weiter behandelt, die Probelösung zugesetzt und erneut gewaschen. Schließlich wird mit 250 mU/ml eines LH-Peroxidase-Konjugats inkubiert (1 Stunde, 37 °C). Es wird erneut gewaschen und die Peroxidase-Aktivität, beispielsweise mit ABTS bestimmt.

Beispiel 3

Bestimmung der Kreuzreaktivität mit anderen Glykoprotein-Hormonen

Es wird, wie in Beispiel 2 beschrieben, vorgegangen. Zunächst wird die Reaktivität von LH bestimmt. Dann wird zu dem jeweiligen monoklonalen Antikörper jeweils das auf Kreuzreaktion zu prüfende Antigen (hCG, TSH, FSH) in steigender Konzentration zugegebenen.

Die Kreuzreaktionen werden anschließend nach folgender Formel berechnet:

$$\frac{C\,(LH)}{C\,(kreuzreagierendes\;Antigen)} \times 100 = \%\;Kreuzreaktion$$

C = Konzentration des Antigens, die zur Erreichung von 50 % des maximalen Signals erforderlich ist.

In der folgenden Tabelle 1 sind die gemessenen Werte für MAK 369 und MAK 799 zusammengestellt.

Tabelle 1:

| Kreuzreaktion der monoklonalen Antikörper MAK 369 und MAK 799 gegen LH mit hCG, FSH und TSH | | | | | |
|---|---|---|---|---|---|
| Glykohormon | Herkunft (Firma) | max. Konz. im Serum | Konzbereich. im Test auf Kreuzreakt. | MAK 369 % Kreuzreakt. | MAK 799 % Kreuzreakt. |
| hCG | UCB, Belgien | 200 IU/ml | 0 - 250 IU/ml | < 0.1 | < 0.1 |
| FSH | UCB, Belgien | 400 mIU/ml | 0 - 9 IU/ml | < 0.1 | < 0.1 |
| $\alpha$-TSH | Boehringer Mannheim | 1000 $\mu$IU/ml | 0 - 9000 $\mu$IU/ml | < 0.1 | < 0.1 |
| $\beta$-TSH | Boehringer Mannheim | 1000 $\mu$IU/ml | 0 - 9000 $\mu$IU/ml | < 0.1 | < 0.1 |

Beispiel 4

Bestimmung der Epitopspezifität

Eine Mikrotiterplatte wird mit 10 $\mu$g/ml Schaf-Antikörper gegen die Fc$\gamma$-Region eines Maus-Antikörpers in 0.2 M Carbonatpuffer, pH 9.6, 2 Stunden bei 37° C oder über Nacht bei 4° C beschichtet. Danach wird mit PBS/Tween 20 (0,1 %), pH 7.35 gewaschen. Anschließend werden 100 $\mu$l eines monoklonalen Antikörpers (MAK 1, Konzentration 10 $\mu$g/ml) in Inkubationspuffer (PBS, 0.1 % Rinderserumalbumin (RSA), 0,1 % Tween 20) zugegeben und 2 Stunden bei 37° C inkubiert.

Ein LH-Peroxidase-Konjugat (100 mU/ml) wird mit 10 $\mu$g/ml eines zweiten monoklonalen Antikörpers (MAK 2) in Lösung bei 4° C über Nacht vorinkubiert.

Nach der Inkubation der Platte mit MAK 1 wird überschüssiger MAK 1 durch Waschen mit PBS-Tween 20 entfernt. Die Platte wird darauf mit 1 % Maus-Normalserum in PBS-RSA 30 Minuten bei Raumtemperatur nachbeschichtet. 100 $\mu$l des vorinkubierten MAK 2/LH-Peroxidase-Komplexes wird auf die Platte gegeben und 2 Stunden bei 37° C inkubiert. Die gebundene Peroxidase-Aktivität wird mit ABTS als Substrat sichtbar gemacht. Die gemessene Farbintensität ist direkt proportional dem an MAK 1 gebundenen MAK 2/LH-Peroxidase-Konjugats. Für MAK 799 als MAK 1 und MAK 369 als MAK 2 beträgt die gebundene Aktivität 34 % der nicht kompetitierten LH-POD-Aktivität..

Die gefundenen Ergebnisse zeigen, daß die beiden monoklonalen Antikörper gegen verschiedene Epitope des Antigens LH gerichtet sind

Beispiel 5

Bestimmung von LH

A) Herstellung der Reagenzlösungen

1) Substratpuffer:

15 mM Natrium-Phosphat-Puffer, pH 7,4
15 mM Natriumchlorid
5 mM EDTA
0,2 % Rinderserumalbumin, pH 7,4
5 mM o-Nitrophenylgalactosid

2) Rezeptor-1-Lösung:
Als Rezeptor 1 wird ein erfindungsgemäßer monoklonaler Maus-anti-hLH-Antikörper eingesetzt. Die diesen Antikörper enthaltende Ascitesflüssigkeit wird ad 1.8 M mit Ammoniumsulfat versetzt. Das Präzipitat wird in einem Puffer am 15 mM Natriumphospnat, pH 7,0 und 50 mM Natriumchlorid aufgenommen. Die so erhaltene Lösung wird einer Passage über DEAE-Cellulose unterworfen.

3) Rezeptor-3-Lösung:

Als Rezeptor 3 wird ebenfalls ein erfindungsgemäßer monoklonaler Maus-Anti-hLH-Antikörper eingesetzt, der jedoch eine andere antigene Determinante als Rezeptor 1 erkennt. Die diesen Antikörper enthaltende Ascitesflüssigkeit wird, wie unter 2) angegeben, aufgereinigt. Der vollständige Antikörper wird in bekannter Weise zum Fab-Fragment gespalten. Die erhaltenen Fab-Fragmente werden mit β-Galactosidase gekoppelt nach der Methode von R. R. Porter, Biochem. J. <u>73</u>, (1959) S. 119.

4) aktivierter Rezeptor-2-Lösung:

Schaf-anti-Maus-Fcγ-Antiserum wird ad 1,8 M mit Ammoniumsulfat versetzt. Das Präzipitat wird in einem Puffer aus 15 mM Natriumphosphat, pH 7,0 und 50 mM Natriumchlorid aufgenommen. Die so erhaltene Lösung wird einer Passage über DEAE-Zellulose unterworfen.

B) Herstellung von Reagenzträgern

1) Reagenzträger 1:

40 μl einer Lösung, die pro ml
100 μmol Natriumphosphat pH 7,3 (37° C),
2 μmol Magnesiumchlorid,
0,9 % Natriumchlorid,
0,5 % Rinderserumalbumin,
5 μg/ml Anti-hLH-monoklonale Antikörper aus Maus (Rezeptor-1-Lösung),
100 mU Anti-hLH-Antikörper-(Maus)-Fab-Fragment-β-Galactosidase-Konjugat (Rezeptor-3-Lösung)

enthält. Wird auf ein Vlies aufgetropft, das aus kommerziellem Polyesterpapier besteht. Anschließend wird bei Raumtemperatur getrocknet. Diese Vliese werden bis zu ihrer Verwendung bei 4° C und einer relativen Luftfeuchtigkeit von 20 % aufbewahrt.

2) Reagenzträger 2:

Auf ein Zellulose-Vlies werden nach dem bekannten Bromcyan-Aktivierungsverfahren (DE-OS 1768512) Schaf-Antikörper gegen den Fcγ-Teil von Maus-Antikörpern (aktivierter Rezeptor-2-Lösung) fixiert, wobei pro g Fasermaterial 10 μg Antikörper zur Fixierung angeboten werden. Durch Waschen wird ungekoppelter Antikörper entfernt und das Vlies schonend bei Raumtemperatur getrocknet. Die Lagerung der so erhaltenen Vliese folgt analog Reagenzträger 1.

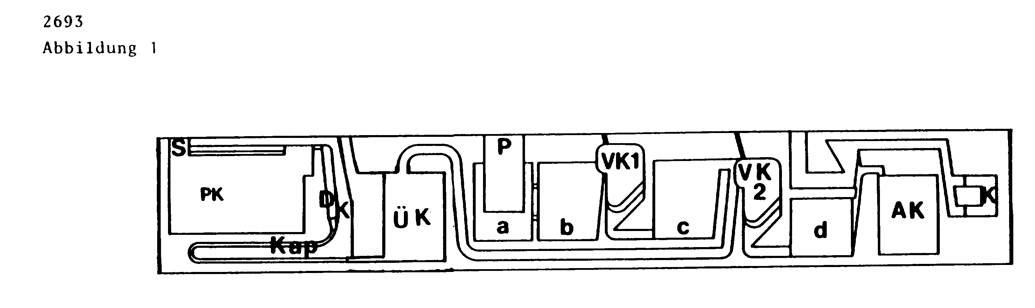

Die Bestimmung mit Hilfe dieser beiden Reagenzträger 1 und 2 erfolgt unter Zuhilfenahme der in der Deutschen Patentanmeldung 3425008.5 beschriebenen Vorrichtung zur Durchführung analytischer Bestimmungen. Diese offenbart ein Rotoreinsatzelement für Zentrifugalanalysenautomaten, bestehend aus einem Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial weiter außen führt, wenn das Einsatzelement auf dem Rotor befestigt ist und weiter wenigstens eine weitere Kammer für die Aufnahme einer Flüssigkeit und einen Transportweg, der von dieser Kammer zur Meßkammer führt und mit dem Probenflüssigkeitstransportweg mindestens teilweise identisch ist, aufweist. Dabei führt der Probenflüssigkeitstransportweg von einer Probenauftragskammer (P) über eine mit saugfähigem Material gefüllte, Puffer enthaltende Kammer (a), eine Kammer (c) und eine zwischen den Kammern (a) und (c) angeordnete erste Ventilkammer (VK1) zu einer zweiten Ventilkammer (VK2) und von dieser über die Kammer (d) und über eine Auffangkammer (AK) zur Meßkammer (K). Zur Aufnahme einer weiteren Flüssigkeit ist eine als Pumpenkammer ausgebildete Substratkammer (PK) vorgesehen, welche über eine aus Dosierkammer (DK) und Kapillare (Kap) bestehende Dosiereinrichtung und eine Überlaufkammer (ÜK) mit der zweiten Ventilkammer (VK2) verbunden ist. Abb. 1 zeigt schematisch das verwendete Rotoreinsatzelement. Hierbei wird Reagenzträger 1 auf Feld c des Disposables plaziert und Reagenzträger 2 auf Feld d. Dabei werden 40 μl Probe durch eine Öffnung am oberen Rand direkt auf das Feld a pipettiert. Die Probe ist unverdünnt. 270 μl Substratlösung werden in Kammer PK pipettiert. Durch ein geeignetes Programm, wo hohe Drehzahlen mit Stillstand abwechseln, werden dann Probe und Substratlösung in Richtung Trennmatrix und Küvette gefördert.

Dabei werden im Verlauf des Programms die Rezeptoren 1 und 3 durch die Probenflüssigkeit vom Feld c eluiert und die homogene Mischung anschließend zur Reaktion gebracht. Auf Feld d werden die gebildeten Komplexe an den Rezeptor 2 gebunden. Der Transfer der Probe von Feld c nach d erfolgt innerhalb sehr Kurzer Zeit.

Die Substratlösung wird durch die Dosierkammer DK in Portionen geteilt, von denen die ersten zum Auswaschen von überschüssigem, nicht komplexiertem Konjugat dienen.

Die über Komplexbildung auf d gebundene β-Galactosidase-Aktivität ist proportional zur in der Probe enthaltenen hLH-Menge. Diese Aktivität wird mit einem weiteren Substratschluck bestimmt, wobei das Substrat in einer 5minütigen Reaktion zu farbigen Produkten umgesetzt wird. Die gebildete Farbe wird in der Küvette bei 410 nm gemessen.

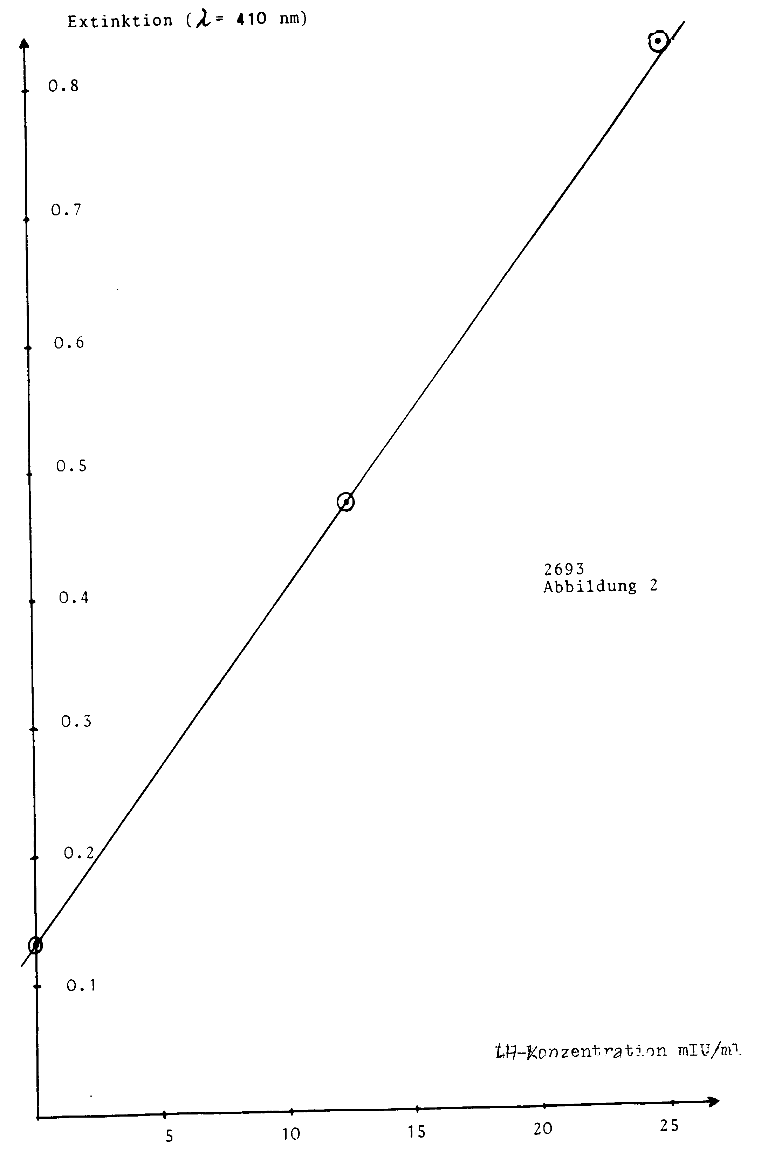

Von Eichseren mit bekanntem LH-Gehalt wird die Eichkurve gemäß Abbildung 2 erhalten, die den Bereich von 0 bis 25 mU hLH/ml (standardisiert nach dem ersten IRP-Standard für hLH 68/40) erfaßt und eine ausreichend empfindliche Messung hLH in Serum oder Plasma ermöglicht. Anhand dieser Eichkurve kann der unbekannte Gehalt hLH in Körperflüssigkeiten, beispielsweise Serum oder Probe ermittelt werden. Die Wiederfindung von mit 100 IU/ml hCG (1. IRP = 1. internationale Referenzpräpation der WHO) aufgestockten Standards beträgt 96 - 104 %.

Beispiel 6

Bestimmung von LH nach dem Sandwich-Prinzip

100 µl einer Lösung, die einen erfindungsgemäßen monoklonalen Antikörper gegen LH im Beschichtungspuffer (0,2 M Natriumcarbonat/-Bicarbonat, pH 9,4) in einer Konzentration von 50 µg/ml enthält, werden in jede Vertiefung einer Mikrotiterplatte gegeben und für eine Stunde bei Raumtemperatur inkubiert. Anschließend wird mit Inkubationspuffer (1 % Rinderserumalbumin, 0,9 % NaCl) nachbeschichtet und 30 Minuten bei Raumtemperatur inkubiert. Nach Waschen mit Waschpuffer (0,9 % NaCl, 0,1 % Tween 20) werden in jede Vertiefung 100 µl Probe, die das zu bestimmende LH enthält, gegeben und 30 Minuten bei Raumtemperatur inkubiert. Nach nochmaligem Waschen mit Waschpuffer wird mit 100 µl eines Konjugates aus Peroxidase (Aktivität 100 mU/ml) und einem weiteren erfindungsgemäßen monoklonalen Antikörper, der jedoch gegen ein anderes Epitop von LH gerichtet ist , beladen und 1 Stunde bei Raumtemperatur inkubiert.

Zur Herstellung des Konjugats wird Meerrettich-Peroxidase EC 1.11.1.7. verwendet. Das Konjugat wird hergestellt durch Oxidation mit Perjodat und anschließender Reduktion mit Borhydrid, entsprechend der Vorschrift von P. K. Nakane (M. B. Wilson und P. K. Nakane, in W. Knapp ed. "Immunofluorescense and Related Staining Techniques", 1978, Elsevier/North Holland, Biomedical Press, Seiten 215 - 224).

Nach Waschen mit Waschpuffer wird mit 100 µl ABTS-Substratlösung beladen und nach einer Stunde Farbreaktion die Extinktion bei 405 nm in einem ELISA-Reader (Dynatec) gemessen.

Zur Herstellung einer Eichkurve werden bei den vorstehend beschriebenen Verfahren anstelle der Probe Lösungen eingesetzt, die LH in verschiedenen, definierten Konzentrationen enthalten.

**Patentansprüche**

1. Monoklonaler Antikörper gegen das luteinisierende Hormon (LH), der eine Kreuzreaktivität mit FSH, TSH und hCG von weniger als 3 % aufweist.

2. Monoklonaler Antikörper gemäß Anspruch 1, dadurch gekennzeichnet, daß er eine Kreuzreaktivität mit FSH, TSH und hCG von weniger als 1 % aufweist.

3. Immunologisches Verfahren zur Bestimmung des luteinisierenden Hormons, dadurch gekennzeichnet, daß mindestens ein monoklonaler Antikörper gemäß Anspruch 1 verwendet wird.

4. Verfahren gemäß Anspruch 3, dadurch gekennzeichnet, daß mindestens ein monoklonaler Antikörper gemäß Anspruch 1 verwendet wird, der gegen LH gerichtet ist und mit FSH, TSH und hCG weniger als 1 % kreuzreagiert.

5. Verfahren gemäß einem der Ansprüche 3 oder 4, dadurch gekennzeichnet, daß mindestens zwei gegen LH gerichtete monoklonale Antikörper eingesetzt werden, von denen mindestens einer ein monoklonaler Antikörper gemäß Anspruch 1 ist.

6. Reagenz zur Bestimmung des luteinisierenden Hormons, dadurch gekennzeichnet, daß es mindestens einen monoklonalen Antikörper gemäß Anspruch 1 enthält.

7. Reagenz gemäß Anspruch 6, dadurch gekennzeichnet, daß es mindestens einen monoklonalen Antikörper enthält, der gegen LH gerichtet ist und mit FSH, TSH und hCG weniger als 1 % kreuzreagiert.

8. Reagenz gemäß einem der Ansprüche 6 oder 7, dadurch gekennzeichnet, daß es mindestens zwei gegen LH gerichtete monoklonale Antikörper enthält, von denen mindestens einer ein monoklonaler Antikörper gemäß Anspruch 1 ist.

9. Verfahren zur Gewinnung eines monoklonalen Antikörpers nach einem der Ansprüche 1 oder 2, dadurch gekennzeichnet, daß man geeignete Tiere mit LH immunisiert, aus der Milz der immunisierten Tiere B-Lymphozyten gewinnt, diese mit Myelomazellen fusioniert, die gebildeten Hybridoma-Zellen kloniert, Klone, die spezifisch gegen LH gerichtete Antikörper produzieren, selektiert und die durch sie gebildeten Antikörper gewinnt.

10. Hybridoma-Zellinie, die monoklonale Antikörper gemäß Anspruch 1 produziert.

11. Hybridoma-Zellinien mit den Hinterlegungsnummern NCACC 84122001 und NCACC 84122005.


## Claims

1. Monoclonal antibody against the luteinizing hormone (LH) which has a cross-reactivity with FSH, TSH and hCG of less than 3 %.

2. Monoclonal antibody as claimed in claim 1, wherein it has a cross-reactivity with FSH, TSH and hCG of less than 1 %.

3. Immunological method for the determination of the luteinizing hormone, wherein at least one monoclonal antibody as claimed in claim 1 is used.

4. Method as claimed in claim 3, wherein at least one monoclonal antibody as claimed in claim 1 is used which is directed against LH and cross-reacts with FSH, TSH and hCG by less than 1 %.

5. Method as claimed in one of the claims 3 or 4, wherein at least two monoclonal antibodies directed against LH are used of which at least one is a monoclonal antibody as claimed in claim 1.

6. Reagent for the determination of luteinizing hormone, wherein it contains at least one monoclonal antibody as claimed in claim 1.

7. Reagent as claimed in claim 6, wherein it contains at least one monoclonal antibody which is directed against LH and cross-reacts with FSH, TSH and hCG by less than 1 %.

8. Reagent as claimed in one of the claims 6 or 7, wherein it contains at least two monoclonal antibodies directed against LH of which at least one is a monoclonal antibody as claimed in claim 1.

9. Process for the isolation of a monoclonal antibody as claimed in one of the claims 1 or 2, wherein suitable animals are immunized with LH, B-lymphocytes are isolated from the spleen of the immunized animals, these are fused with myeloma cells, the hybridoma cells which form are cloned, clones that produce specific antibodies directed against LH are selected and the antibodies produced by them are isolated.

10. Hybridoma cell line which produces monoclonal antibodies as claimed in claim 1.

11. Hybridoma cell lines with the deposit numbers NCACC 84122001 and NCACC 84122005.


## Revendications

1. Anticorps monoclonal contre l'hormone lutéinisante (LH) qui présente une réactivité croisée avec FSH, TSH et hCG inférieure à 3%.

2. Anticorps monoclonal selon la revendication 1, caractérisé en ce qu'il présente une réactivité croisée avec FSH, TSH et hCG inférieure à 1%.

3. Procédé immunologique pour la détermination de l'hormone lutéinisante, caractérisé en ce qu'au moins un anticorps monoclonal selon la revendication 1 est utilisé.

4. Procédé selon la revendication 3, caractérisé en ce qu'au moins un anticorps monoclonal selon la revendication 1 qui est dirigé contre LH et qui réagit de manière croisée avec FSH, TSH et hCG à raison de moins de 1% est utilisé.

5. Procédé selon l'une des revendications 3 ou 4, caractérisé en ce qu'au moins deux anticorps monoclonaux dirigés contre LH, parmi lesquels l'un au moins est un anticorps monoclonal selon la revendication 1, sont utilisés.

6. Réactif pour la détermination de l'hormone lutéinisante, caractérisé en ce qu'il contient au moins un anticorps monoclonal selon la revendication 1.

7. Réactif selon la revendication 6, caractérisé en ce qu'il contient au moins un anticorps monoclonal qui est dirigé contre LH et qui réagit de manière croisée avec FSH, TSH et hCG à raison de moins de 1%.

8. Réactif selon l'une des revendications 6 ou 7, caractérisé en ce qu'il contient au moins deux anticorps monoclonaux dirigés contre LH parmi lesquels l'un au moins est un anticorps monoclonal selon la revendication 1.

9. Procédé pour obtenir un anticorps monoclonal selon l'une des revendications 1 ou 2, caractérisé en ce que l'on immunise des animaux appropriés avec LH, on obtient à partir de la rate des animaux immunisés des lymphocytes B, on fusionne ceux-ci avec des cellules de myélome, on clone les cellules d'hybridome formées, on sélectionne des clones qui produisent des anticorps dirigés spécifiquement contre LH et on obtient les anticorps formés par eux.

10. Lignée cellulaire d'hybridome qui produit des anticorps monoclonaux selon la revendication 1.

11. Lignées cellulaires d'hybridome ayant les numéros de dépôt NCACC 84122001 et NCACC 84122005.

2693
Abbildung 1

Extinktion ($\lambda$ = 410 nm)

2693
Abbildung 2

LH-Konzentration mIU/ml